# EUROPÄISCHE PATENTSCHRIFT

(11) **EP 0 612 496 B1**
(45) Veröffentlichungstag und Bekanntmachung des Hinweises auf die Patenterteilung: **09.10.1996**
(21) Anmeldenummer: 94100075.4
(22) Anmeldetag: 05.01.1994
(51) Int. Cl.: A61B 1/00

(54) **Schaft für medizinische Instrumente**
Handle for medical instruments
Poignée pour instruments médicaux

(30) Priorität: 22.02.1993 DE 4305376
(43) Veröffentlichungstag der Anmeldung: 31.08.1994
(73) Patentinhaber: Richard Wolf GmbH, 75438 Knittlingen (DE)
(72) Erfinder: Heckele, Helmut, D-75438 Knittlingen (DE)
(74) Vertreter: Wilcken, Thomas, Dipl.-Ing.

(56) Entgegenhaltungen:
- DE-A- 3 602 092
- US-A- 5 174 277
- US-A- 5 179 935

## Beschreibung

Die Erfindung geht aus von einem Schaft für medizinische Instrumente, insbesondere zum Führen von Instrumenten in eine Körperhöhle, bei dem zwecks Einstellung von Schaftkrümmungen benachbarte, innen hohle und Schaftabschnitte bildende Segmente in ihrer Position mittels Steuerdrähten verstellbar sind, wobei mindestens zwei Gruppen von Segmenten unabhängig voneinander verstellbar sind, wobei mindestens zwei Gruppen von Segmenten unabhängig von einander verstellbar sind u. die Segmente einer ersten, distal vorgesehenen Gruppe gelenkig miteinander verbunden sind. Ein derartiger Schaft ist aus der US-A-5 179 935 bekannt.

Bei medizinischen Eingriffen, bei denen ein Instrument in eine Körperhöhle eingeführt werden muß, ist es häufig sinnvoll, das Instrument durch einen flexiblen Schaft zu führen, da in vielen Fällen eine Behandlung sonst schwer möglich ist und die Anwendung von flexiblen Instrumenten dem Patienten weniger Schmerzen verursacht als die Anwendung von starren Instrumenten. So ist es beispielsweise bei der endoskopischen Diagnose und Therapie häufig erforderlich, zumindest das distale Ende der verwendeten Instrumente in der Körperhöhle in verschiedene Richtungen biegen und zudem in dieser gebogenen Position belassen zu können.

Beispielsweise ist es nach der DE - AS 1 019 048 bekannt, einen dünnwandigen Instrumentenschaft vor der Benutzung in die gewünschte Form zu biegen und dann zum Beispiel in eine Körperhöhle einzuführen. Nachteilig an einem derartigen Instrument ist jedoch, daß es nur begrenzt einsetzbar ist, da die einmal vor Benutzung eingestellte Position während der Behandlung nicht mehr verändert werden kann.

Weiterhin sind auch biegsame Rohranordnungen und Schäfte zur Verwendung in einem Endoskop, beispielsweise speziell auf dem Gebiet der Bronchoskopie, bekannt, bei denen die Rohranordnung zumindest teilweise Bereiche von aneinandergereihten, gegeneinander beweglichen Segmenten aufweist, wobei die Segmente durch Steuerdrähte, welche zu einer Handhabe geführt sind, zwecks Einstellung von Schaftkrümmungen bewegt werden können, wie es beispielsweise das DE-U- 6938 905, die US-PSen 3 190 286, US-PS 5 179 935, die DE-AS 1 291 437 und die DE-OSen 1 766 209, 1 816 973 und 1 950 035 darlegen. Bei der DE-AS 1 816 973 sind die einzelnen Segmente des Schaftes über Gabeln miteinander verbunden, die bei zusammengefügten Bauteilen ineinandergreifen. Eine derartige Lösung wirkt sich als nachteilig aus, da die Gabeln der Segmente leicht brechen oder verformt werden können.

Diese Lösungen sind unterschiedlich gut bei der Endoskopie einsetzbar, sie haben aber alle den Nachteil, daß im verspannten Zustand, d.h. wenn die Krümmung des distalen Schaftendes festgelegt wurde und mit Instrumenten eine Manipulation in der Körperhöhle vorgenommen werden soll, keine wirkliche Starrheit des Schaftes erreichbar ist und der Schaft aufgrund der unvermeidbaren Krafteinwirkungen des eingeführten Insrumentes auf ihn leicht von der gewünschten Lage abweichen kann.

Mit diesen Schäften sind somit die Eigenschaften eines starren Schaftes nicht erreichbar, während vorgebogene Schäfte, wie aus der DE-AS 1 019 048 bekannt, nur beschränkt einsetzbar sind, da sie während der Benutzung die zuvor eingestellte Form beibehalten. Deshalb ist die Lösung gemäß der DE-OS 1 766 209 insgesamt besser, bei welcher die Gruppen aus einzelnen Segmenten unabhängig voneinander mittels Drähten betätigt werden können. Die eingestellte Position kann jedoch bei dieser Lösung nicht fixiert werden.

Die Aufgabe der Erfindung besteht darin, einen Schaft für medizinische Instrumente, insbesondere zum Führen von Instrumenten in eine Körperhöhle, anzugeben, dessen Krümmung bei der Einführung in eine Körperhöhle und während der Untersuchung einer Körperhöhle leicht verändert werden kann und außerdem so in einer Arbeitsposition eingestellt, d.h. fixiert werden kann, daß er wie ein starrer Schaft handhabbar ist.

Diese Aufgabe wird erfindungsgemäß dadurch gelöst, daß die sich proximalwärts anschließenden Segmente der zweiten Gruppe kraftschlüssig mit einer variabel einstellbaren Federkraft verspannbar u. durch gegenseitigen Eingriff formschlüssig verbindbar sind.

Der Vorteil dieser erfindungsgemäßen Lösung besteht darin, daß die Segmente der Gruppen unabhängig voneinander eingestellt werden können. Zudem kann vor und während der Anwendung in einer Körperhöhle der distale Schaftteil eingestellt bzw. verstellt werden, so daß er stets den Gegebenheiten angepaßt werden kann, während die Segmente des proximalen Schaftteiles so stark verspannt werden können, daß dieser Schaftteil starr ist.

Weiterhin sind die Segmente der zweiten Gruppe mit Spanndrähten zusammengehalten, welche in vorteilhafter Weise in einer Spanneinrichtung innerhalb der Handhabe des Instrumentes festgelegt sind. Aufgrund der erfinderischen Lösung werden infolge einer besonderen Ausführungsform der Segmente diese bei sehr starker Verspannung über einen Formschluß und nicht mehr, wie bei geringerer Verspannung, über einen Kraftschluß zusammengehalten.

Innerhalb der Handhabe sind außer der Spanneinrichtung Verstelleinrichtungen für die Segmentgruppen vorgesehen, so daß eine unabhängige Verstellung der beiden Gruppen vorgenommen werden kann. Die Verstellung der einzelnen Segmente erfolgt über Steuerdrähte, welche die Segmente durchlaufen. Für jede Gruppe sind jeweils zwei Steuerdrähte vorgesehen, die in den jeweiligen Verstelleinrichtungen in der Handhabe enden. Je nach Anordnung der Steuerdrähte innerhalb der Segmente beider Gruppen kann schließlich der gesamte Schaft entweder in einer oder in zwei Ebenen bewegt werden. Außerdem können mehrere Gruppen mit unterschiedlichen Segmenten hintereinandergereiht werden, so daß der Schaft auf mehreren Abschnitten unterschiedlich gekrümmt werden kann. Folglich sollte dann auch die Handhabe entsprechend mit Verstelleinrichtungen ausgerüstet sein.

Vorteilhafte Weiterbildungen der Erfindung sind durch weitere in den Unteransprüchen aufgeführten Merkmale gekennzeichnet.

Die Erfindung wird nachfolgend anhand eines in der Zeichnung dargestellten Ausführungsbeispieles näher erläutert. Es zeigen:
- Figur 1: eine Gesamtansicht eines erfindungsgemä ßen Schaftes mit eingeführtem Instru ment,
- Figur 2: eine Teilansicht des Schaftes,
- Figuren 3 bis 6: Schnitte gemäß der Linien III-III, IV-IV, V-V, VI-VI in Figur 2,
- Figur 7: einen Abschnitt des Schaftes nach Figur 1 im Schnitt,
- Figur 8: einen anderen Abschnitt des Schaftes nach Figur 1 im Schnitt,
- Figur 9: eine Spanneinrichtung für Schaftsegmente,
- Figuren 10 bis 12: Ausführungsbeispiele für Schaftsegmente in Seitenansicht,

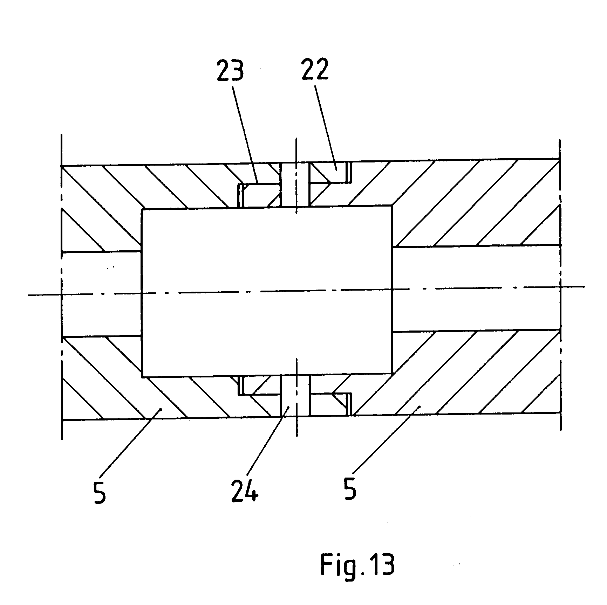

- Figur 13: einen Schnitt durch den Schaft im Bereich von zwei Segmenten und
- Figur 14: eine Einrichtung zum Verspannen von Schaftsegmenten.

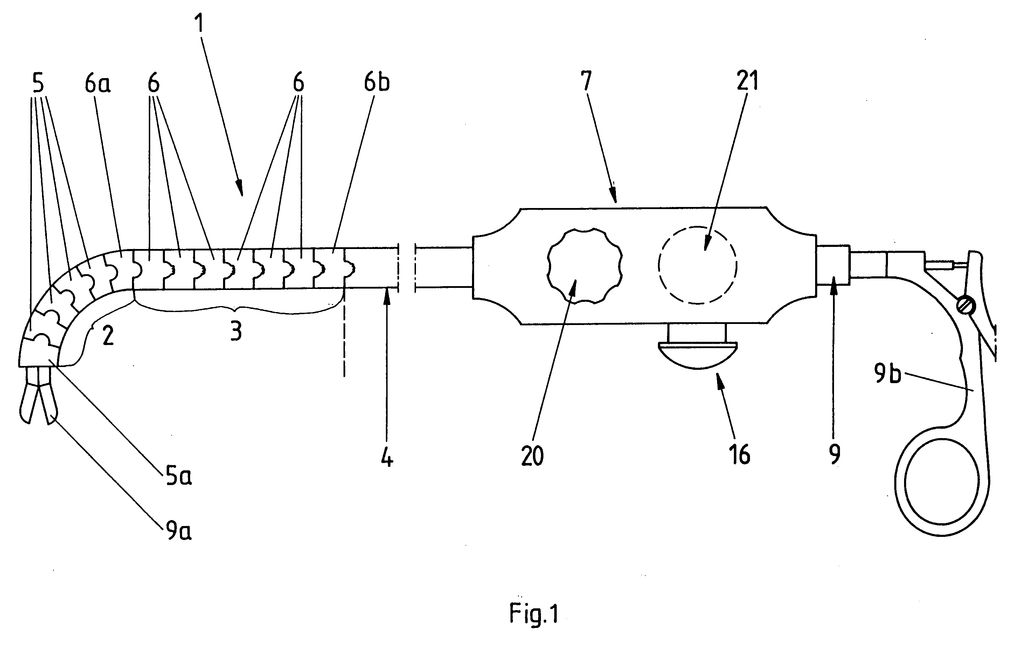

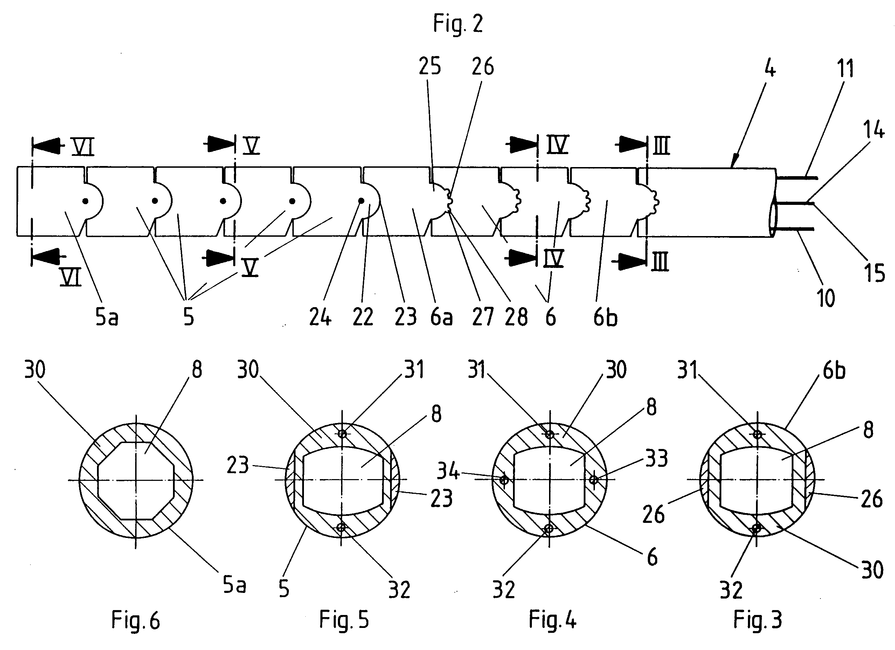

Bei dem Ausführungsbeispiel nach Figur 1 weist der erfindungsgemäße Schaft 1 in seinem Verlauf drei verschiedene Schaftabschnitte auf, welche innen hohl sind. Proximal ist eine Handhabe 7 vorgesehen. Den ersten Schaftabschnitt stellt eine erste, distale Segmentgruppe 2 aus einzelnen Segmenten 5 dar. Die Segmente 5 sind gelenkig miteinander verbunden, und sie sind mittels Steuerdrähten 10, 11 zwecks Einstellung von Schaftkrümmungen in ihrer Position verstellbar. An die erste, distale Gruppe 2 schließt sich proximalwärts eine zweite Segmentgruppe 3 an, die ebenfalls aus einzelnen Segmenten 6 besteht. Diese Segmente 6 unterscheiden sich in ihrer Bauart von denen der ersten Gruppe 2, worauf noch bei der Beschreibung der Figuren 2,7 und 8 eingegangen wird. Die Segmente 6 der zweiten Gruppe 3 sind kraftschlüssig miteinander verbunden und mit einer variabel einstellbaren Federkraft verspannt. Die Segmente der beiden Gruppen 2 und 3 sind unabhängig voneinander verstellbar. Außerdem können die Segmente 5, 6 von einem nicht dargestellten flexiblen Schlauch als äußere Hülle umgeben sein.

Es versteht sich, daß die Anzahl der Gruppen nicht, wie in diesem Ausführungsbeispiel dargestellt, auf zwei beschränkt sein muß. Es besteht auch die Möglichkeit, mehrere Gruppen von Segmenten hintereinander vorzusehen, welche unabhängig voneinander verstellt werden können, wodurch der Schaft auf drei oder mehr Abschnitten unterschiedlich gekrümmt werden kann. Unabhängig von der Anzahl der Gruppen schließt sich an die proximal letzte Gruppe ein Schaftabschnitt an, in diesem Ausführungsbeispiel ist es der Schaftteil 4, welcher in üblicher Weise starr ausgeführt ist und in der Handhabe 7 endet. Durch das Innenlumen 8 des Schaftes 1 kann ein Instrument 9 zur Behandlung des Patienten hindurchgeführt werden. In Figur 1 ist ein solches Instrument 9 durch sein distales Ende 9a und seine Handhabe 9b angedeutet.

Figur 1 stellt weiterhin die Handhabe 7 des Schaftes 1 dar, welche die verschiedenen Einrichtungen zur Ein- und Verstellung und zur Verspannung der Segmente 5 und 6 der Gruppen 2 und 3 enthält. Auch die Handhabe 7 ist in bekannter Weise innen hohl, um das Instrument 9 hindurchführen zu können. Die Handhabe 7 weist eine Verstelleinrichtung 20 auf, welche zur Einstellung der Schaftkrümmung im Bereich der ersten, distalen Gruppe 2 mittels der Steuerdrähte 10 und 11 dient. Analog hierzu ist eine zweite Verstelleinrichtung 21, wie in Figur 1 gestrichelt dargestellt, vorgesehen, die für die Verstellung der Segmente 6 der zweiten Gruppe 3 mittels Steuerdrähten 12 und 13 dient. Werden mehr Gruppen von Segmenten vorgesehen, die unabhängig voneinander in ihrer Position verstellt werden sollen, so können entsprechend viele Verstelleinrichtungen in der Handhabe 7 vorgesehen werden. Schließlich weist die Handhabe 7 noch eine Spanneinrichtung 16 zur Fixierung der jeweiligen Positionen der Segmente 6 auf, die mit Spanndrähten 14 und 15 zusammengehalten werden.

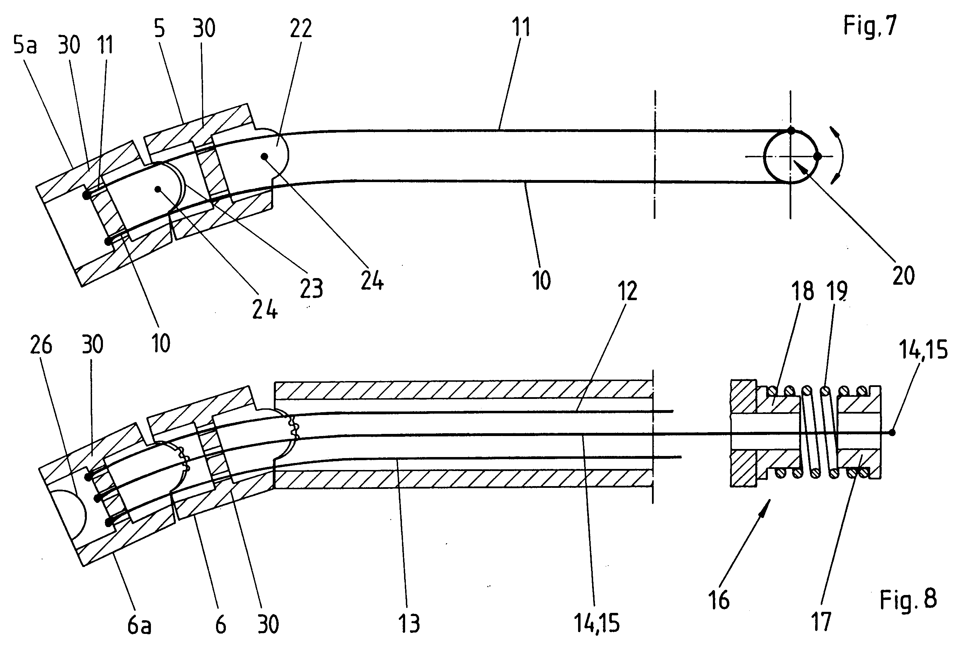

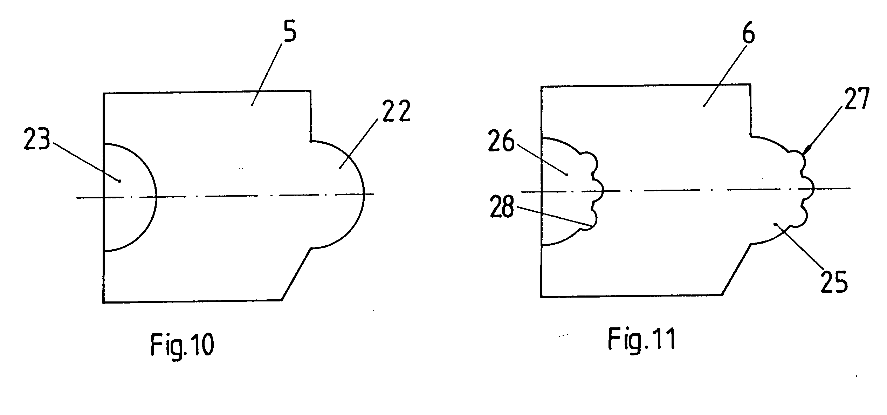

Die Figuren 2 bis 8 und 10 bis 12 verdeutlichen die Ausführungsformen der unterschiedlich ausgebildeten Segmente 5 und 6. Die Segmente 5 der Gruppe 2 weisen einenendes halbkreisförmige Vorsprünge 22 und anderenendes ebensolche Ausnehmungen 23 auf, die sich jeweils diametral, wie im Schnitt gemäß Figur 7 und in Figur 10 dargestellt, gegenüberliegen. Derart ausgebildete Segmente 5 können aufgrund ihrer Form zu einem Schaftabschnitt zusammengefügt werden, indem jeweils die beiden Vorsprünge 22 eines Segmentes 5 in die korrespondierenden Ausnehmungen 23 eines nächstfolgenden Segementes 5 eingefügt werden. Zwei Segemente 5 werden jeweils seitlich über Verbindungsteile 24, beispielsweise Stifte oder Nieten, achsgelenkig miteinander verbunden. Das distal letzte Segment 5a, wie in Figur 2 ersichtlich, kann mit einer ebenen Fläche abgeschlossen werden, welche das distale Ende des Schaftes 1 bildet. Weiterhin sind an diesem letzten Segment 5a die Steuerdrähte 10 und 11 festgelegt, wie es Figur 7 verdeutlicht.

Ähnlich den Segmenten 5 der ersten Gruppe 2 weisen auch die Segmente 6 der zweiten Gruppe 3 einenendes halbkreisförmige Vorsprünge 25 und anderenendes Ausnehmungen 26 auf, die sich jeweils diametral entsprechend den Figuren 2 und 11 gegenüberliegen. Die Konturen der Vorsprünge 25 und der Ausnehmungen 26 sind wellenlinienförmig, so daß bei zusammengefügten Segmenten 6 stets Wellenberge 27 der Vorsprünge 25 eines Segmentes 6 in Wellentäler 28 der Ausnehmungen 26 eines nächstfolgenden Segmentes 6 eingreifen. Alternativ oder zusätzlich hierzu besteht die Möglichkeit, die Ausnehmungen der Segmente 6 in gleicher Weise wie die der Segmente 5 der ersten Gruppe 2 auszubilden und in der Mitte der Bogenlinie einer jeden Ausnehmung 26 einen Raststift vorzusehen, so daß bei zusammengefügten Segmenten 6 jeder Vorsprung 25 eines Segmentes 6 mit dem Raststift eines nächstfolgenden Segmentes 6 zusammenwirkt.

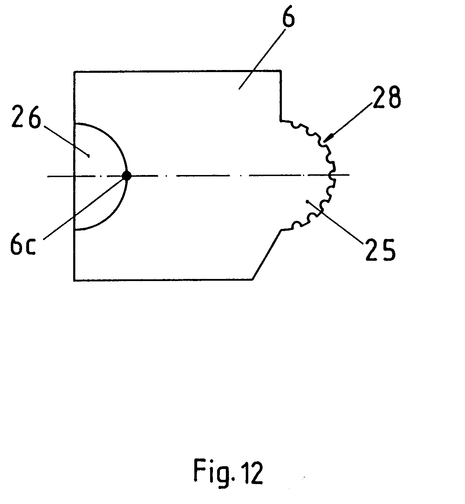

Eine weitere Möglichkeit zur Verrastung der Segmente 6 mit Raststiften zeigt die Figur 12, bei der die Segmente 6 an einem Ende mit wellenlinienartigen Vorsprüngen 25 und am anderen Ende mit Raststiften 6c ausgestattet sind. Somit können die Raststifte 6c eines Segmentes mit den Vorsprüngen 25 eines anderen Segmentes zusammenarbeiten, indem je nach Stellung der beiden Segmente zueinander die Raststifte in eines der Wellentäler 28 des jeweils benachbarten Segmentes fassen.

Das distal letzte Segment 6a der zweiten Gruppe 3 ist achsgelenkig, beispielsweise mittels Nieten 24, mit dem proximalen ersten Segment 5 der ersten Gruppe 2 verbunden. Zur Aufnahme der Vorsprünge 25 des proximal letzten Segmentes 6b ist das distale Ende des starren Schaftteiles 4 mit Ausnehmungen 26 entsprechend denen der Segmente 6 versehen und weist je nach Ausführungsform einen Raststift auf oder ist wellenlinienförmig ausgebildet.

Die Länge einer jeden schaftbildenden Gruppe, in diesem Ausführungsbeispiel die Gruppen 2 und 3, kann nach den gegebenen Behandlungsbedingungen, d.h. nach Einsatzort und Art der einzusetzenden Instrumente bestimmt werden, indem entsprechend viele Segmente 5 bzw. 6 aneinandergereiht werden.

Figur 7 zeigt den Verlauf der Steuerdrähte 10 und 11 der Segmente 5, welche sich proximalwärts bis zu der Verstelleinrichtung 20 fortsetzten und dort festgelegt sind. Die Steuerdrähte 10 und 11 verlaufen durch Bohrungen 31 und 32 innerhalb der Wand 30 der Segmente 5 und 6 beider Gruppen 2 und 3. Verstelleinrichtungen dieser Art sind bekannt, so daß diese z.B. wie in der DE-OS 1 766-209 oder als einfache Winde nach der US-PS 3 190 286 ausgeführt sein können. Gleiches gilt für die Verstelleinrichtung 21. Auf eine Beschreibung einer solchen Verstelleinrichtung wird daher verzichtet.

Auch die Segmente 6 der zweiten Gruppe 3 werden mit Steuerdrähten 12 und 13 verstellt, welche gemäß Figur 8 am Segment 6a festgelegt und analog zu den Steuerdrähten 10 und 11 der Segmente 5 in Bohrungen der Segmente 6 bis hin zu der weiteren Verstelleinrichtung 21 verlaufen und geführt sind. Die Steuerdrähte 12 und 13 können z.B. zusammen mit den Steuerdrähten 10 und 11 oder zusammen mit den Spanndrähten 14 und 15 in den jeweiligen Bohrungen 31 und 32 bzw. 33 und 34 geführt werden, so daß keine zusätzlichen Bohrungen nötig sind. Verlaufen die Steuerdrähte 12 und 13 zusammen mit den Steuerdrähten 10 und 11 in den Bohrungen 31 und 32, so ist eine Verstellung des gesamten Schaftes 1 in einer Ebene möglich. Beim Verlauf der Steuerdrähte 12 und 13 in den Bohrungen 33 und 34 ist die Verstellung des gesamten Schaftes 1 in zwei Ebenen möglich.

Die durch die Bohrungen 33 und 34 der Segmente 6 verlaufenden Spanndrähte 14 und 15 sind proximal in einer Spanneinrichtung 16 festgelegt, während die distalen Enden der Spanndrähte 14 und 15 am Segment 6a befestigt sind.

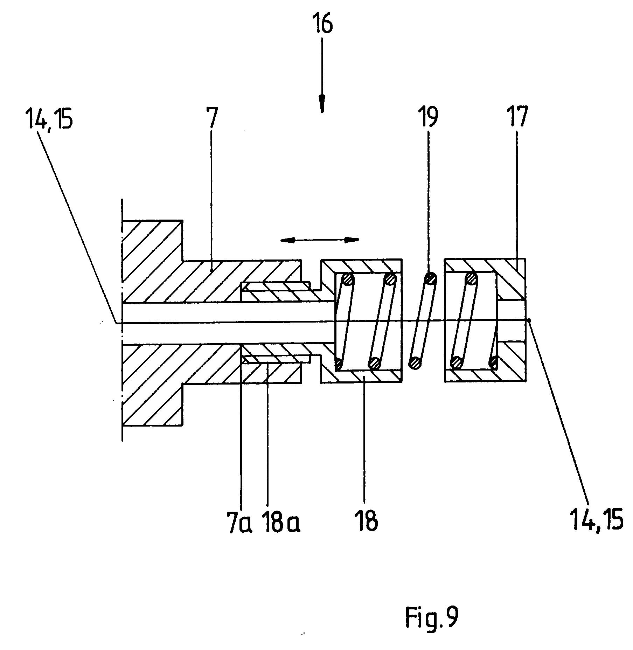

Die Spanneinrichtung 16, wie sie anhand der Figur 9 dargestellt ist, kann in einfacher Weise aus zwei Aufnahmen 17 und 18 für eine Feder 19, beispielsweise eine Schraubenfeder, bestehen, die als Druckfeder die beiden Aufnahmen 17 und 18 im Abstand zueinander hält. Die Spanndrähte 14 und 15 werden durch die beiden Aufnahmen 17 und 18 und die Feder 19 hindurchgeführt und an der proximalen Aufnahme 17 festgelegt. Die distale Aufnahme 18 weist einen mit Gewinde ausgebildeten Bereich 18a auf, über dessen Verstellung innerhalb eines Gegengewindes 7a der Handhabe 7 eine Einstellung der Verspannung der Segmente 6 erfolgt, indem der Abstand der Aufnahmen und damit die Federspannung und die Zugkraft der die Segmente 6 zusammenhaltenden Spanndrähte 14, 15 verändert werden.

Unter der Federbelastung führt das Ineinandergreifen der Vorsprünge und Ausnehmungen der Segmente 6 zu einer kraftschlüssigen Verbindung, wobei aber immer noch eine Bewegung der Segmente 6 gegeneinander und gegen den starren Schaftteil 4 unter zeitweiliger Aufhebung der Rastverbindungen zwischen den unter Federverspannung verbleibenden Segmenten möglich ist. Der durch die Segmente 6 gebildete Schaftabschnitt kannn so beispielsweise von Hand vor dem Einführen in eine Körperhöhle vorgeformt werden, wobei dann anschließend durch Verstellen und Erhöhen des Federdruckes vermittels der Spanneinrichtung 16 ein vollständig starrer Schaftabschnitt erreicht werden kann, während der durch die Segmente 5 gebildete Schaftabschnitt verstellbar bleibt.

Weiterhin besteht die Möglichkeit, den Schaft 1 im geraden Zustand völlig starr festzulegen, an den Ort der Behandlung zu bringen und dort dann in bezug auf seine Krümmung den Gegebenheiten anzupassen. Hierzu wird vorab der Federdruck, wie oben beschrieben, zur Festlegung der Segmente 6 der zweiten Gruppe 3 eingestellt, und es werden die Steuerdrähte 10 und 11 festgezogen. Somit kann ein insgesamt starrer Schaft 1 erzielt werden. In der Körperhöhle kann dann der starre Zustand durch Umkehrung des vorstehenden Vorgehens aufgehoben werden.

Dabei können die Segmente 5 der ersten Gruppe 2 mit den Steuerdrähten 10 und 11 zwecks Einstellung der benötigten Schaftkrümmung verstellt werden. Bei einer entsprechend starken Krümmung der ersten, distalen Gruppe 2 können sich die Segmente 6 unter Überwindung des durch die Federbelastung bewirkten Kraftschlusses an der Krümmung beteiligen und in eine neue Lage einrasten. Als vorteilhaft erweist sich jedoch eine unabhängige Verstellmöglichkeit der Segmente 6 über die dann zusätzlich vorzusehenden Steuerdrähte 12 und 13.

Werden die Spanndrähte 14 und 15 für den Zusammenhalt der Segmente 6 mittels der Spanneinreichtung 16 über das Verstellen der Aufnahme 18 innerhalb der Handhabe 7 extrem stark verspannt, so greifen Wellenberge 27 der Vorsprünge 25 eines Segmentes 6 in Wellentäler 28 der Ausnehmungen 26 des nächsten folgenden Segmentes 6 derart ein, daß die Segmente 6 über einen Formschluß und nicht wie bei geringerer Verspannung nur durch einen Kraftschluß zusammengehalten werden. Entsprechendes gilt natürlich auch bei der alternativ möglichen Ausführungsform der Segmente 6 mit Raststiften.

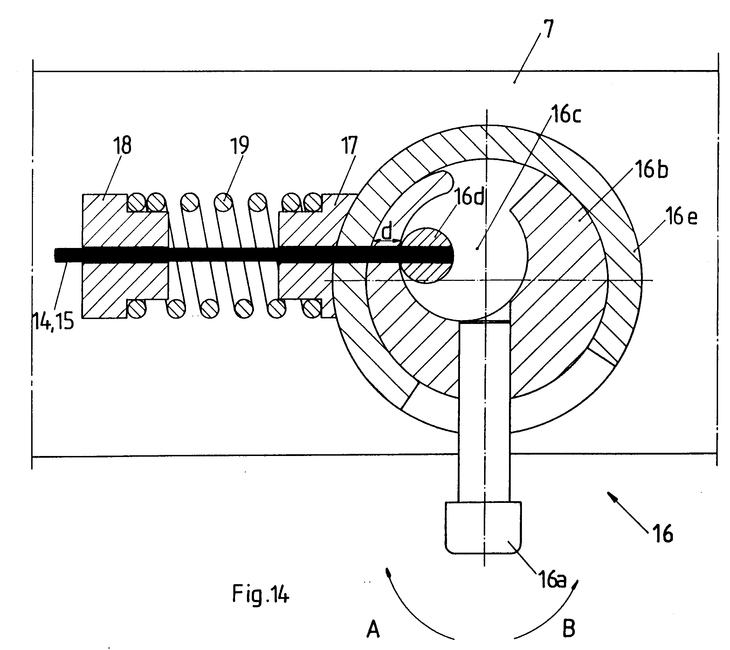

Eine einfach und besonders schnell durchführbare Verspannung der Segmente 6 läßt sich mit der in Figur 14 gezeigten Spanneinrichtung erreichen, bei der gleiche oder gleichwirkende Teile der Spanneinrichtungen gemäß den Figuren 8 und 9 zur Anwendung kommen, weshalb diese Teile und die Spanneinrichtung in Figur 14 mit den gleichen entsprechenden Bezugszeichen versehen sind.

Mit der innerhalb der Handhabe 7 angebrachten Spanneinrichtung 16 gemäß Figur 14 können die durch die Feder 19 vorgespannten Spanndrähte 14, 15 so stark verspannt werden, daß der Schaftteil aus den Segmenten 6 praktisch starr wird. Hierzu wird über einen aus der Handhabe herausgeführten Verstellhebel 16a eine Scheibe 16b mit exzentrischer Bohrung 16c, innerhalb der die Enden der Spanndrähte z. B. mit einer Kugel 16d gefaßt sind, innerhalb eines Gehäuses 16e verstellt bzw. verdreht. Bei Bewegung des Verstellhebels 16a in Richtung A, also zum distalen Schaftende hin, werden auf Grund der sich vergrößernden Strecke d die Spanndrähte proximalwärts gezogen und die Segmente 6 verspannt, während umgekehrt, also beim Bewegen des Verstellhebels 16a in Richtung B, die Spanndrähte entspannt und die die Segmente 6 zusammenhaltenden Kräfte reduziert werden.

## Patentansprüche

1. Schaft (1) für medizinische Instrumente, insbesondere zum Führen von Instrumenten (9) in eine Körperhöhle, bei dem zwecks Einstellung von Schaftkrümmungen benachbarte, innen hohle und Schaftabschnitte bildende Segmente (5, 6) in ihrer Position mittels Steuerdrähten (10, 11; 12,13) verstellbar sind, wobei mindestens zwei Gruppen (2, 3) von Segmenten (5, 6) unabhängig voneinander verstellbar sind und die Segmente (5) einer ersten, distal vorgesehenen Gruppe (2) gelenkig miteinander verbunden sind, dadurch gekennzeichnet, daß die sich proximalwärts anschließenden Segmente (6) der zweiten Gruppe (3) kraftschlüssig mit einer variabel einstellbaren Federkraft verspannbar und durch gegenseitigen Eingriff formschlüssig verbindbar sind.

2. Schaft nach Anspruch 1, dadurch gekennzeichnet, daß die Segmente (6) der zweiten Gruppe (3) mit Spanndrähten (10, 11) zusammengehalten sind, deren distale Enden am distalen Segment (6a) der zweiten Gruppe (3) befestigt sind, während die Spanndrähte (14, 15) proximalwärts durch einen starren Schaftteil (4) verlaufen und die proximalen Spanndrahtenden in einer Spanneinrichtung (16) innerhalb einer Handhabe (7) fixiert sind.

3. Schaft nach Anspruch 2, dadurch gekennzeichnet, daß die Spanneinrichtung (16) aus zwei Aufnahmen (17, 18) für eine Feder (19) besteht, welche die beiden Aufnahmen (17, 18) im Abstand zueinander hält, wobei die distale Aufnahme (18) einen mit Gewinde ausgebildeten Bereich (18a) aufweist, welcher in ein Gegengewinde (7a) der Handhabe (7) eingedreht ist, daß die proximalen Enden der Spanndrähte (14, 15) an der proximalen Aufnahme (17) befestigt sind und daß die distalen Enden der durch Bohrungen in den Segmenten (6) der zweiten Gruppe (3) geführten Spanndrähte (14, 15) am distal letzten Segment (6a) dieser Gruppe festgelegt sind.

4. Schaft nach einem der Ansprüche 1 bis 3, dadurch gekennzeichnet, daß die Segmente (5, 6) beider Gruppen (2, 3) mit Steuerdrähten (10, 11; 12, 13) über zwei Verstelleinrichtungen (20, 21) innerhalb der Handhabe (7) verstellbar sind.

5. Schaft nach einem der Ansprüche 1 bis 4, dadurch gekennzeichnet, daß die Segmente (5) der ersten Gruppe (2) einenendes zwei kreisförmige Vorsprünge (22) und anderenendes ebensolche Ausnehmungen (23) aufweisen, die sich jeweils diametral gegenüberliegen, und daß jeweils die Vorsprünge (22) eines Segmentes (5) in die korrespondierenden Ausnehmungen (23) eines nächsfolgenden Segmentes (5) eingefügt und die beiden Segmente (5) achsgelenkig miteinander verbunden sind.

6. Schaft nach einem der Ansprüche 2 bis 4, dadurch gekennzeichnet, daß an dem distalseitig letzten Segment (5a) der ersten Gruppe (2) Steuerdrähte (10, 11) mit ihren distalen Enden festgelegt sind.

7. Schaft nach einem der Ansprüche 1 bis 6, dadurch gekennzeichnet, daß die Segmente (6) der zweiten Gruppe (3) einenendes halbkreisförmige, sich diametral gegenüberliegende, Vorsprünge (25) aufweisen, deren Umfang wellenlinienförmig ausgebildet ist, und anderenendes ebensolche Ausnehmungen (26) aufweisen, wobei Wellenberge (27) der Vorsprünge (25) eines Segmentes (6) stets in Wellentäler (28) der Ausnehmungen (26) eines nächstfolgendes Segmentes (6) eingreifen.

8. Schaft nach einem der Ansprüche 1 bis 6, dadurch gekennzeichnet, daß die Segmente (6) der zweiten Gruppe (3) einenendes im wesentlichen halbkreisförmige, sich jeweils diametral gegenüberliegende Vorsprünge (25) aufweisen, deren Umfang wellenlinienförmig ausgebildet ist, und anderenendes halbkreisförmige Ausnehmungen (26) aufweisen, wobei Raststifte (29) eines jeden Segmentes (6), welche jeweils in der Mitte der Bogenlinie einer jeden Ausnehmung (26) angebracht sind, in die wellenlinienförmigen Vorsprünge (25) eines nächstfolgenden Segmentes (6) eingerastet sind.

9. Schaft nach Anspruch 7 oder 8, dadurch gekennzeichnet, daß das distal letzte Segment (6a) der zweiten Gruppe (3) im Bereich seiner Ausnehmungen (26) mit den Vorsprüngen (22) des folgenden Segmentes (5) der ersten Gruppe (2) achsgelenkig verbunden sind.

10. Schaft nach einem der Ansprüche 7 bis 9, dadurch gekennzeichnet, daß das distale Ende eines proximalen starren Schaftteiles (4) mit Ausnehmungen (26) zur Aufnahme der Vorsprünge (25) des proximal letzten Segmentes (6b) der zweiten Gruppe (3) versehen ist.

## Claims

1. A shaft (1) for medical instruments, in particular for guiding instruments (9) into a body cavity, in which for the purpose of adjusting curvatures in the shaft, adjacent, internally hollow segments (5, 6) forming shaft sections are adjustable in their position by means of control wires (10, 11; 12, 13), in which at least two groups (2, 3) of segments (5, 6) are adjustable independently of each other and the segments (5) of a first, distally provided group (2) are connected articulatedly with each other, characterised in that the segments (6) of the second group (3), adjoining each other in proximal direction, are able to be braced in a force-fitting manner with a variably adjustable elastic force and are able to be connected in a form-fitting manner through mutual engagement.

2. A shaft according to Claim 1, characterised in that the segments (6) of the second group (3) are held together with bracing wires (10, 11), the distal ends of which are fastened to the distal segment (6a) of the second group (3), whilst the bracing wires (14, 15) run in a proximal direction through a rigid shaft piece (4) and the proximal bracing wire ends are feed in a bracing device (16) inside a handle (7).

3. A shaft according to Claim 2, characterised in that the bracing device (16) consists of two mountings (17, 18) for a spring (19), which holds the two mountings (17, 18) at a distance from each other, in which the distal mounting (18) has a region (18a) constructed with a thread, which region (18a) is turned into a counter-thread (7a) of the handle (7), that the proximal ends of the bracing wires (14, 15) are fastened to the proximal mounting (17) and that the distal ends of the bracing wires (14, 15), guided through bores in the segments (6) of the second group (3), are secured on the distally last segment (6a) of this group.

4. A shaft according to one of Claims 1 to 3, characterised in that the segments (5, 6) of the two groups (2, 3) are adjustable by control wires (10, 11; 12, 13) via two adjusting devices (20, 21) inside the handle (7).

5. A shaft according to one of Claims 1 to 4, characterised in that the segments (5) of the first group (2) have at one end two circular projections (22) and at the other end equivalent recesses (23), which in each case lie diametrically opposite each other, and that in each case the projections (22) of a segment (5) are fitted into the corresponding recesses (23) of a following segment (5) and the two segments (5) are connected with each other in an axially articulated manner.

6. A shaft according to one of Claims 2 to 4, characterised in that control wires (10, 11) are fastened by their distal ends on the last segment (5a) - on the distal side - of the first group (2).

7. A shaft according to one of Claims 1 to 6, characterised in that the segments (6) of the second group (3) have at one end semicircular projections (25) lying diametrically opposite each other, the circumference of which is constructed in an undulating shape, and at the other end have equivalent recesses (26), in which undulation peaks (27) of the projections (25) of a segment (6) always engage in undulation troughs (28) of the recesses (26) of a following segment (6).

8. A shaft according to one of Claims 1 to 6, characterised in that the segments (6) of the second group (3) at one end have substantially semicircular projections (25) lying diametrically opposite each other in each case, the circumference of which is constructed in an undulating shape, and at the other end have semicircular recesses (26), in which detent pins (29) of each segment (6), which are arranged in each case in the centre of the arc line of each recess (26), are engaged into the undulating projections (25) of a following segment (6).

9. A shaft according to Claim 7 or 8, characterised in that the distally last segment (6a) of the second group (3) in the region of its recesses (26) is connected in an axially articulated manner with the projections (22) of the following segment (5) of the first group (2).

10. A shaft according to one of Claims 7 to 9, characterised in that the distal end of a proximal rigid shaft piece (4) is provided with recesses (26) to receive the projections (25) of the proximally last segment (6b) of the second group (3).

## Revendications

1. Corps en forme de tige (1) pour instruments médicaux, notamment destiné à guider des instruments (9) dans une cavité du corps, dans lequel, en vue de régler des courbures du corps en forme de tige, des segments (5, 6) voisins, intérieurement creux, et formant des tronçons du corps en forme de tige, peuvent être ajustés dans leur position, au moyen de fils de commande (10, 11; 12, 13), au moins deux groupes (2, 3) de segments (5, 6) pouvant être ajustés indépendamment l'un de l'autre, et les segments (5) d'un premier groupe (2) prévu côté distal étant reliés les uns aux autres de manière articulée, caractérisé en ce que les segments (6) du second groupe (3), se raccordant côté proximal, peuvent être serrés par adhérence, à l'aide d'une force élastique variable, réglable, et peuvent être reliés par complémentarité de forme par engrènement réciproque.

2. Corps en forme de tige selon la revendication 1, caractérisé en ce que les segments (6) du second groupe (3) sont maintenus assemblés au moyen de fils de serrage (14, 15) dont les extrémités distales sont fixées au segment distal (6a) du second groupe (3), tandis que les fils de serrage (14, 15) s'étendent, côté proximal, à travers une partie rigide (4) du corps en forme de tige, et les extrémités proximales des fils de serrage sont immobilisées dans un dispositif de serrage (16) à l'intérieur d'une poignée (7).

3. Corps en forme de tige selon la revendication 2, caractérisé en ce que le dispositif de serrage (16) est constitué de deux éléments de réception (17, 18) pour un ressort (19), qui maintient les deux éléments de réception (17, 18) à distance l'un de l'autre, l'élément de réception distal (18) comportant une zone (18a) pourvue d'un filetage, qui est vissée dans un filetage conjugué (7a) de la poignée (7), en ce que les extrémités proximales des fils de serrage (14, 15) sont fixées à l'élément de réception proximal (17), et en ce que les extrémités distales des fils de serrage (14, 15), guidés à travers des forures dans les segments (6) du second groupe (3), sont fixées au dernier segment (6a), côté distal, de ce groupe.

4. Corps en forme de tige selon l'une des revendications 1 à 3, caractérisé en ce que les segments (5, 6) des deux groupes (2, 3) peuvent être ajustés à l'aide de fils de commande (10, 11; 12, 13), par l'intermédiaire de deux dispositifs de réglage (20, 21) à l'intérieur de la poignée (7).

5. Corps en forme de tige selon l'une des revendications 1 à 4, caractérisé en ce que les segments (5) du premier groupe (2) comportent, à une extrémité, deux protubérances (22) de forme circulaire et, à l'autre extrémité, des évidements (23) de même nature, qui sont diamétralement opposés l'un à l'autre, et en ce que les protubérances (22) d'un segment (5) sont engagées dans les évidements (23) correspondants d'un segment (5) immédiatement suivant, et les deux segments (5) considérés sont reliés l'un à l'autre de manière articulée autour d'un axe.

6. Corps en forme de tige selon l'une des revendications 2 à 4, caractérisé en ce qu'au dernier segment (5a), côté distal, du premier groupe (2), sont fixés, par leurs extrémités distales, des fils de commande (10, 11).

7. Corps en forme de tige selon l'une des revendications 1 à 6, caractérisé en ce que les segments (6) du second groupe (3) comportent, à une extrémité, des protubérances (25) en forme de demi-cercle, diamétralement opposées l'une à l'autre et dont la périphérie présente une forme ondulée, et, à l'autre extrémité, des évidements (26) de même nature, des crêtes d'onde (27) des protubérances (25) d'un segment (6) venant s'engager continuellement dans des creux d'onde (28) des évidements (26) d'un segment (6) immédiatement suivant.

8. Corps en forme de tige selon l'une des revendications 1 à 6, caractérisé en ce que les segments (6) du second groupe (3) comportent, à une extrémité, des protubérances (25) sensiblement en forme de demi-cercle, diamétralement opposées l'une à l'autre et dont la périphérie présente une forme ondulée, et, à l'autre extrémité, des évidements (26) en forme de demi-cercle, des crans d'encliquetage (29) de chacun des segments (6), qui sont placés chacun au milieu de l'arc de cercle de chaque évidement (26), étant encliquetés dans les protubérances (25) de forme ondulée du segment (6) immédiatement suivant.

9. Corps en forme de tige selon la revendication 7 ou 8, caractérisé en ce que le dernier segment (6a), côté distal, du second groupe (3), dans la zone de ses évidements (26), est relié de manière articulée autour d'un axe, aux protubérances (22) du segment suivant (5) du premier groupe (2).

10. Corps en forme de tige selon l'une des revendications 7 à 9, caractérisé en ce que l'extrémité distale d'une partie proximale rigide (4) du corps en forme de tige, est pourvue d'évidements (26) destinés à recevoir les protubérances (25) du dernier segment (6b), côté proximal, du second groupe (3).
